# EUROPEAN PATENT APPLICATION

(11) **EP 4 643 844 A1**
(43) Date of publication of application: **05.11.2025**
(21) Application number: 23911906.8
(22) Date of filing: 20.12.2023
(51) Int. Cl.: A61K 8/44, A61K 8/60, A61K 8/365, A61Q 11/00

(54) **ORAL COMPOSITION**

(30) Priority: 27.12.2022 JP 2022209241
(71) Applicant: Sunstar Inc., Takatsuki-shi Osaka 569-1195 (JP); Sunstar Suisse SA, 1163 Etoy (CH)
(72) Inventor: SUEKAWA Yutaka, Takatsuki-shi, Osaka 569-1195 (JP); PESARO Manuel, 1163 Etoy (CH)
(74) Representative: Acapo Onsagers AS
(86) International application number: PCT/JP2023/045792
(87) International publication number: WO 2024/143126

(57) **Abstract**

Provided is an oral composition containing a specific sugar, an amino acid and citric acid and/or an analog thereof. The specific sugar is a polysaccharide containing, as constituent sugars, a single galactose and one or a plural number of sugars other than galactose. The citric acid and/or an analog thereof is at least one selected from citric acid and citric acid salts. The content of the specific sugar is 0.5-5 mass% inclusive. The content of the amino acid is 0.5-4 mass% inclusive. The content of the citric acid and/or an analog thereof is 0.001-2 mass% inclusive.

## Description

### TECHNICAL FIELD

The present disclosure relates to an oral composition.

### BACKGROUND ART

Streptococcus oralis (hereinafter also referred to as *S. oralis*) is known as a kind of non-pathogenic bacterium among bacteria in the oral cavity. *S. oralis* belongs to the mitis group of the genus Streptococcus. Other bacterial species belonging to the mitis group are also classified as non-pathogenic bacteria, like *S. oralis.*

Patent Literature 1 discloses an oral composition that selectively suppresses growth of pathogenic bacteria without affecting growth of bacterial species belonging to the mitis group of the genus Streptococcus. That is, the oral composition has a selective antibacterial action against pathogenic bacteria in the oral cavity. Improving balance of bacterial flora in the oral cavity by increasing a proportion of non-pathogenic bacteria in the bacterial flora in the oral cavity is useful for preventing oral diseases and suppressing the progression thereof.

### CITATION LIST

### Patent Literature

Patent Literature 1: JP 2016-113460 A

### SUMMARY OF INVENTION

### Technical Problem

It has been suggested that increasing a concentration of S. *oralis* in the oral cavity is useful, in terms of ensuring a healthy oral environment. The present inventors have found that a combination of a specific sugar and an amino acid contributes to proliferation of *S. oralis.* However, when a sugar and an amino acid are used in combination, a Maillard reaction occurs in which the sugar and the amino acid react with each other to produce a brown substance. Therefore, a composition having both a sugar and an amino acid is likely to undergo discoloration due to the Maillard reaction.

### Solution to Problem

Aspects of the present disclosure are described.

### [Aspect 1]

An oral composition comprising a specific sugar, an amino acid, and a citric acid compound, wherein the specific sugar is a polysaccharide including a single galactose as a constituent sugar, the citric acid compound is at least one selected from citric acid and a citrate salt, a content of the specific sugar is 0.5 mass% or more and 5 mass% or less, a content of the amino acid is 0.5 mass% or more and 4 mass% or less, and a content of the citric acid compound is 0.001 mass% or more and 2 mass% or less.

### [Aspect 2]

The oral composition according to [Aspect 1], wherein the specific sugar is a non-reducing sugar.

### [Aspect 3]

The oral composition according to [Aspect 2], wherein
the amino acid is at least one selected from a basic amino acid compound and an acidic amino acid compound,
the basic amino acid compound is at least one selected from a basic amino acid and a basic amino acid salt, and
the acidic amino acid compound is at least one selected from an acidic amino acid and an acidic amino acid salt.

### [Aspect 4]

The oral composition according to [Aspect 3], wherein
the basic amino acid compound is arginine,
the acidic amino acid compound is at least one selected from glutamic acid, a glutamate salt, aspartic acid, and an aspartate salt, and
the non-reducing sugar is raffinose.

### [Aspect 5]

The oral composition according to [Aspect 2], wherein the amino acid is a neutral amino acid.

### [Aspect 6]

The oral composition according to [Aspect 5], wherein
the neutral amino acid is citrulline, and
the non-reducing sugar is raffinose.

### [Aspect 7]

The oral composition according to [Aspect 1], wherein the specific sugar is lactose, and the amino acid is at least one acidic amino acid compound selected from an acidic amino acid and an acidic amino acid salt.

### [Aspect 8]

The oral composition according to [Aspect 7], wherein the acidic amino acid compound is at least one selected from glutamic acid, a glutamate salt, aspartic acid, and an aspartate salt.

### [Aspect 9]

The oral composition according to any one of [Aspect 1] to [Aspect 7], which is used as an oral composition for bacterial proliferation that promotes proliferation of S. oralis.

### DESCRIPTION OF EMBODIMENTS

Hereinafter, an oral composition of the present embodiment will be specifically described.

The oral composition of the present embodiment is applied as an oral composition for bacterial proliferation that aims at, for example, improving balance of bacterial flora in the oral cavity, specifically, promoting proliferation of *S. oralis.*

The oral composition of the present embodiment comprises a specific sugar, an amino acid, and a citric acid compound.

A pH of the oral composition is, for example, 5.5 or more and 9.0 or less, and preferably 6.0 or more and 8.5 or less.

### Specific Sugar

The specific sugar is a polysaccharide including a single galactose as a constituent sugar. In other words, the specific sugar is a heteropolysaccharide including a single galactose and a single additional sugar or a plurality of additional sugars other than galactose as constituent sugars. In the specific sugar, the constituent sugars are bonded to each other via a glycosidic bond. The number of monosaccharides bonded in the specific sugar is, for example, 2 or more and 5 or less, and preferably 2 or more and 4 or less. The specific sugar is, for example, a disaccharide or a trisaccharide. The specific sugar may be an oligosaccharide in which the number of bonded monosaccharides is from 4 to 9, or may be a polysaccharide in which the number of bonded monosaccharides is 10 or more.

Examples of the constituent sugar other than galactose included in the specific sugar include hexoses, such as glucose, fructose, and fucose, and pentoses, such as xylose and ribose. The constituent sugar other than galactose is preferably a hexose.

Examples of the specific sugar include a reducing sugar, such as lactose, and a non-reducing sugar, such as raffinose. Lactose is a disaccharide composed of one galactose and one glucose as constituent sugars. Raffinose is a trisaccharide composed of one fructose, one galactose, and one glucose as constituent sugars. The oral composition may comprise only one of the specific sugars described above alone, or may comprise two or more thereof in combination.

The reducing sugar is a sugar having a free aldehyde group or ketone group, or a hemiacetal-bonded aldehyde group or ketone group. The non-reducing sugar means a saccharide that does not have a free reducing group, as opposed to the reducing sugar. That is, the non-reducing sugar means a saccharide having none of free aldehyde group and ketone group and hemiacetal-bonded aldehyde group and ketone group.

A content of the specific sugar in the oral composition is 0.5 mass% or more and 5 mass% or less. The content of the specific sugar is preferably 1 mass% or more, more preferably 1.5 mass% or more. The content of the specific sugar is preferably 4 mass% or less, more preferably 3 mass% or less.

The oral composition may comprise an additional sugar that does not correspond to the specific sugar. In this case, the content of the additional sugar in the oral composition is preferably, for example, 5 mass% or less.

### Amino Acid

The amino acid included in the oral composition is not particularly limited. Examples of the amino acid include basic amino acids, such as lysine, arginine, and histidine, and basic amino acid salts (hereinafter basic amino acids and basic amino acid salts are collectively referred to as basic amino acid compounds), acidic amino acids, such as aspartic acid and glutamic acid, and acidic amino acid salts (hereinafter acidic amino acids and acidic amino acid salts are collectively referred to as acidic amino acid compounds), and neutral amino acids, such as alanine, proline, threonine, serine, valine, glycine, and citrulline. Specific examples of the basic amino acid salt include hydrochloride and sulfate salts. Specific examples of the acidic amino acid salt include alkali metal salts, such as a sodium salt and a potassium salt, and alkaline earth metal salts, such as a magnesium salt and a calcium salt. The oral composition may comprise only one of the amino acids described above alone, or may comprise two or more thereof in combination.

A content of the amino acid in the oral composition is 0.5 mass% or more and 4 mass% or less. The content of the amino acid is preferably 1 mass% or more, more preferably 1.5 mass% or more. The content of the amino acid is preferably 3.5 mass% or less, more preferably 3 mass% or less. In the oral composition, a mass ratio of the amino acid to the specific sugar (amino acid/specific sugar) is, for example, 0.05 or more and 10 or less, and preferably 0.25 or more and 4 or less.

### Combination of Specific Sugar and Amino Acid

A preferred combination of the specific sugar and the amino acid is a combination of a non-reducing sugar and an amino acid. In this case, the type of amino acid is not particularly limited, and may be any of basic amino acid compounds, acidic amino acid compounds, and neutral amino acids. Specific examples of the combination of the non-reducing sugar and the amino acid include a combination of raffinose and arginine, a combination of raffinose and glutamic acid, a combination of raffinose and a glutamate salt, a combination of raffinose and aspartic acid, a combination of raffinose and an aspartate salt, and a combination of raffinose and citrulline.

Another preferred combination of the specific sugar and the amino acid is a combination of lactose and an acidic amino acid compound. Specific examples of the combination include a combination of lactose and glutamic acid, a combination of lactose and a glutamate salt, a combination of lactose and aspartic acid, and a combination of lactose and an aspartate salt.

### Citric Acid Compound

The citric acid compound included in the oral composition is at least one selected from citric acid and a citrate salt. Examples of the citrate salt include sodium citrate, magnesium citrate, potassium citrate, and calcium citrate.

A content of the citric acid in the oral composition is 0.001 mass% or more and 2 mass% or less. The content of the citric acid is preferably 0.003 mass% or more, more preferably 0.005 mass% or more. The content of the citric acid is preferably 1.8 mass% or less, more preferably 1.5 mass% or less.

When the amino acid included in the oral composition is a basic amino acid compound, the citric acid compound is preferably citric acid. In this case, in the oral composition, a mass ratio of the citric acid compound to the basic amino acid compound (citric acid compound/basic amino acid compound) is, for example, 0.001 or more and 1.5 or less, and preferably 0.005 or more and 1 or less.

When the amino acid included in the oral composition is an acidic amino acid compound, a mass ratio of the citric acid compound to the acidic amino acid compound (citric acid compound/acidic amino acid compound) in the oral composition is, for example, 0.001 or more and 1 or less, and preferably 0.005 or more and 0.8 or less.

When the amino acid included in the oral composition is a neutral amino acid, a mass ratio of the citric acid compound to the neutral amino acid (citric acid compound/neutral amino acid) in the oral composition is, for example, 0.001 or more and 1 or less, and preferably 0.005 or more and 0.8 or less.

### Application Form, Intended Use, and Dosage Form

An application form of the oral composition is not particularly limited, and the oral composition can be used as, for example, a pharmaceutical product or a quasi-drug. As an intended use of the oral composition, a known intended use can be appropriately adopted, and examples thereof include a toothpaste, a mouthwash, a gargle, a liquid dentifrice, a biofilm dispersant, an oral malodor preventive agent, a gum massage agent, an oral moistening agent, a tongue coating remover, an intraoral application agent, an oral disinfectant, a throat disinfectant, an oral throat agent, a periodontal disease therapeutic agent, a denture mounting agent, a denture coating agent, a denture stabilizer, a denture preservative, a denture cleaner, and an implant care agent.

The dosage form of the oral composition is not particularly limited. For example, due to inclusion of a solvent, such as water or an alcohol, the oral composition can be applied to ointments, pastes, pastas, sprays, gels, liquids, suspensions/emulsifiers, gums, and the like.

The type of water used as the solvent is not particularly limited, and for example, distilled water, pure water, ultrapure water, purified water, tap water, or the like can be used. The type of alcohol used as the solvent is not particularly limited, and ethanol can be used, for example. Water and an alcohol may be used in combination. When the oral composition is constituted in a liquid form, a content of the solvent, such as water, is preferably from 60 to 99.8 mass%, and more preferably from 70 to 95 mass%.

### Additional Component

The oral composition may comprise an additional component besides the components described above depending on the application purpose, form, intended use, and the like. Examples of the additional component include an antibacterial agent, an anti-inflammatory agent, a fragrance, a wetting agent, a surfactant, an abrasive, an alcohol, a thickener, a sweetening component, a medicinal component, a colorant, and a stabilizer. As the additional component, a known component to be blended in the oral composition can be used. The oral composition may comprise only one for each of the additional components described above alone, or may comprise two or more therefor in combination.

Examples of the antibacterial agent include cetylpyridinium chloride, paraben, sodium benzoate, triclosan, chlorhexidine hydrochloride, isopropylmethylphenol, benzalkonium chloride, benzethonium chloride, and hinokitiol.

Examples of the anti-inflammatory agent include allantoin, glycyrrhetinic acid, glycyrrhizinate salts, tranexamic acid, ε-aminocaproic acid, and phellodendron bark extract.

Examples of the fragrance component include menthol, anethole, eugenol, carvone, wintergreen, methyl salicylate, thymol, clove oil, sage oil, ocimene oil, and citronellol.

Examples of the surfactant include a nonionic surfactant, an anionic surfactant, and an amphoteric surfactant.

Examples of the nonionic surfactant include sugar fatty acid esters, such as sucrose fatty acid ester and maltose fatty acid ester, sugar alcohol fatty acid esters, such as maltitol fatty acid ester, sorbitan fatty acid esters, such as sorbitan monolaurate, polyoxyethylene sorbitan fatty acid esters, such as polyoxyethylene sorbitan monolaurate and polyoxyethylene sorbitan monostearate, fatty acid alkanolamides, such as lauric acid diethanolamide, polyoxyethylene alkyl ethers, such as polyoxyethylene stearyl ether and polyoxyethylene oleyl ether, polyethylene glycol fatty acid esters, such as polyethylene glycol monooleate and polyethylene glycol monolaurate, alkyl glucosides, such as lauryl glucoside and decyl glucoside, polyglycerol fatty acid esters, polyoxyethylene glycerol fatty acid esters, polyoxyethylene fatty acid esters, alkyl glucosides, polyoxyethylene hydrogenated castor oil, glycerol fatty acid esters, and polyoxyethylene propylene block copolymers.

Examples of the anionic surfactant include sulfate ester salts, such as sodium lauryl sulfate and sodium polyoxyethylene lauryl ether sulfate, sulfosuccinate salts, such as sodium lauryl sulfosuccinate and sodium polyoxyethylene lauryl ether sulfosuccinate, acylamino acid salts, such as sodium cocoyl sarcosinate and sodium lauroyl methylalanine, and sodium methyl cocoyl taurate.

Examples of the amphoteric surfactant include amino acid-type amphoteric surfactants, such as N-lauryl diaminoethylglycine and N-myristyl diethyl glycine, and betaine-based amphoteric surfactants, such as alkyldimethylaminoacetic acid betaine, N-alkyl-N'-carboxymethyl-N'-hydroxyethyl ethylenediamine salt, 2-alkyl-N-carboxymethyl-N-hydroxyethyl imidazolinium betaine, and cocamidopropyl betaine.

Examples of the abrasive include calcium carbonate, magnesium carbonate, dibasic calcium phosphate, tribasic calcium phosphate, magnesium phosphate, silica, zeolite, sodium metaphosphate, aluminum hydroxide, magnesium hydroxide, calcium pyrophosphate, red iron oxide, calcium sulfate, and silicic anhydride.

Examples of the alcohol include ethyl alcohol, lauryl alcohol, and myristyl alcohol.

Examples of the thickener include sodium polyacrylate, carrageenan, sodium carboxymethylcellulose, sodium alginate, xanthan gum, hydroxyethyl cellulose, hydroxypropyl methylcellulose, methylcellulose, and propylene glycol alginate.

Examples of the medicinal component include fluorides, such as sodium monofluorophosphate, sodium fluoride, stannous fluoride, and strontium fluoride, condensed phosphate salts, such as sodium pyrophosphate and sodium polyphosphate, phosphate salts, such as sodium monohydrogen phosphate and trisodium phosphate, vitamin agents, such as ascorbic acid, sodium ascorbate, pyridoxine hydrochloride, and tocopherol acetate, glucanase enzymes, such as dextranase and mutanase, degrading enzymes, such as protease and lysozyme, inorganic salts, such as zinc chloride, zinc citrate, strontium chloride, potassium nitrate, and aluminum lactate, chelating compounds, such as chlorophyll and glycerophosphate, polyethylene glycol or the like that dissolves fat, sodium chloride, aluminum lactate, and strontium chloride.

Examples of the colorant include legal pigments, such as Green No. 1, Blue No. 1, and Yellow No. 4, and titanium oxide. When the oral composition is transparent or translucent, particularly, in a liquid form, discoloration due to a brown substance produced by a Maillard reaction is likely to become noticeable. Therefore, when the oral composition is in a transparent or translucent liquid form, a discoloration suppressing effect in the present embodiment is particularly remarkably obtained.

Examples of the stabilizer include sodium edetate, sodium thiosulfate, sodium sulfite, calcium lactate, lanolin, triacetin, castor oil, and magnesium sulfate.

### Effects

Next, the actions and effects of the present embodiment will be described.
(1) The oral composition comprises a specific sugar, an amino acid, and a citric acid compound. The specific sugar is a polysaccharide including a single galactose as a constituent sugar. The citric acid compound is at least one selected from citric acid and a citrate salt. The content of the specific sugar is 0.5 mass% or more and 5 mass% or less, the content of the amino acid is 0.5 mass% or more and 4 mass% or less, and the content of the citric acid compound is 0.001 mass% or more and 2 mass% or less.
   According to the above configuration, it is possible to proliferate *S. oralis* in the oral cavity, and to suppress discoloration of the oral composition.
(2) The specific sugar is a non-reducing sugar. According to the above configuration, the discoloration suppressing effect described in the above (1) is more remarkably obtained. In particular, in this case, the Maillard reaction in which the specific sugar and the amino acid react with each other to produce a brown substance is effectively suppressed, and therefore, the discoloration of the oral composition can be more effectively suppressed. In addition, the specific sugar and the amino acid are less likely to be consumed by the Maillard reaction, and thus a decrease in concentration of each of the specific sugar and the amino acid in the oral composition is suppressed. Therefore, the oral composition having the above-described configuration can be stably stored for a long term, in terms of both suppressing discoloration and maintaining an effect of promoting the proliferation of *S. oralis.* The non-reducing sugar is particularly preferably raffinose.
(3) In the configuration described in the above (2), the amino acid is at least one selected from a basic amino acid compound and an acidic amino acid compound. The basic amino acid compound is at least one selected from a basic amino acid and a basic amino acid salt. The acidic amino acid compound is at least one selected from an acidic amino acid and an acidic amino acid salt.
   According to the above configuration, the discoloration suppressing effect described in the above (2) is more remarkably obtained. The basic amino acid compound is particularly preferably arginine. The acidic amino acid compound is particularly preferably at least one selected from glutamic acid, a glutamate salt, aspartic acid, and an aspartate salt.
(4) In the configuration described in the above (2), the amino acid is a neutral amino acid. According to the above configuration, the discoloration suppressing effect described in the above (2) is more remarkably obtained. The neutral amino acid is particularly preferably citrulline.
(5) The specific sugar is lactose. The amino acid is at least one acidic amino acid compound selected from an acidic amino acid and an acidic amino acid salt. According to the above configuration, the discoloration suppressing effect described in the above (1) is more remarkably obtained. The acidic amino acid compound is particularly preferably at least one selected from glutamic acid, a glutamate salt, aspartic acid, and an aspartate salt.

### EXAMPLES

The oral composition of the present disclosure will be described in more detail based on the following Examples and Comparative Examples. The following Examples are non-limiting examples of the oral composition of the present disclosure.

### Preparation of Examples 1 to 15 and Comparative Examples 1 and 2

As Examples 1 to 15 and Comparative Examples 1 and 2, liquid mouthwashes of the formulation example shown in Table 1 were prepared by mixing and stirring the components according to an ordinary method. The types and blended amounts of the sugars, amino acids, and citric acid compounds used in the examples are as shown in Tables 2 to 4. The unit of the numerical values of the components in the tables is mass%.

**[Table 1]**

| Component | Blended amount (mass%) |
|---|---|
| Sugar | 1.5 to 5.0 |
| Amino acid | 1.5 |
| Citric acid compound | 0.01 to 0.71 |
| Water | Balance |
| Total | 100 |

### Long-Term Storage Evaluation

The prepared Examples and Comparative Examples were stored in a sealed state at 55°C for 1 month. Degrees of yellowness of each example were measured before and after the storage treatment, and a rate of change in degree of yellowness (after treatment/before treatment) was determined. The degree of yellowness was measured using an ultraviolet-visible spectrophotometer (UV-2600 type) available from Shimadzu Corporation. Then, based on the rate of change in degree of yellowness, the coloration suppressing effect of each example was evaluated according to the following criteria. The results are shown in Tables 2 to 4.
A: The rate of change in degree of yellowness is 2.0 or less, and the coloration suppressing effect is high.
B: The rate of change in degree of yellowness is more than 2.0 and 4.0 or less, and the coloration suppressing effect is obtained.
C: The rate of change in degree of yellowness is more than 4.0, and the coloration suppressing effect is not obtained.

The pH of each example measured before the storage treatment is shown in Tables 2 to 4.

**[Table 2]**

| | | Comparative Example | | Example | | | | |
|---|---|---|---|---|---|---|---|---|
| | | 1 | 2 | 1 | 2 | 3 | 4 | 5 |
| Sugar | Raffinose | | | 1.5 | 1.5 | 1.5 | 1.5 | 1.5 |
| | Lactose | | | | | | | |
| | Galactose | 1.5 | | | | | | |
| | Galactooligosaccharide | | 1.5 | | | | | |
| Amino acid | Arginine | 1.5 | 1.5 | 1.5 | 1.5 | 1.5 | 1.5 | 1.5 |
| | Na glutamate | | | | | | | |
| | Na aspartate | | | | | | | |
| Citric acid compound | Citric acid | 0.71 | 0.71 | 0.6 3 | 0.5 9 | 0.5 5 | 0.5 2 | 0.5 1 |
| pH | Before treatment | 5.5 | 5.5 | 6 | 6.5 | 7 | 7.5 | 8 |
| Degree of yellowness | Before treatment | 1.9 | 2.9 | 2.0 | 2.2 | 3.0 | 2.1 | 2.1 |
| | After treatment | 146.4 | 65.8 | 2.7 | 2.3 | 2.6 | 2.5 | 2.4 |
| | Rate of change | 77.1 | 22.7 | 1.4 | 1.0 | 0.9 | 1.2 | 1.1 |
| Evaluation | Coloration suppressing effect | C | C | A | A | A | A | A |

**[Table 3]**

| | | Example | | | | | | | |
|---|---|---|---|---|---|---|---|---|---|
| | | 6 | 7 | 8 | 9 | 10 | 11 | 12 | 13 |
| Sugar | Raffinose | 3 | 4 | 5 | 1.5 | 1.5 | | | |
| | Lactose | | | | | | 1.5 | 1.5 | 1.5 |
| | Galactose | | | | | | | | |
| | Galactooligosaccharide | | | | | | | | |
| Amino acid | Arginine | 1.5 | 1.5 | 1.5 | | | 1.5 | | |
| | Na glutamate | | | | 1.5 | | | 1.5 | |
| | Na aspartate | | | | | 1.5 | | | 1.5 |
| Citric acid compound | Citric acid | 0.63 | 0.63 | 0.63 | 0.01 | 0.01 | 0.7 | 0.03 | 0.02 |
| pH | Before treatment | 6 | 6 | 6 | 6 | 6 | 5.5 | 5.5 | 5.5 |
| Degree of yellowness | Before treatment | 2.2 | 2.4 | 2.8 | 2.3 | 2.2 | 2.5 | 2.2 | 2.1 |
| | After treatment | 3.2 | 3.3 | 3.6 | 2.6 | 2.5 | 9.0 | 3.7 | 3.4 |
| | Rate of change | 1.5 | 1.4 | 1.3 | 1.1 | 1.1 | 3.6 | 1.7 | 1.6 |
| Evaluation | Coloration suppressing effect | A | A | A | A | A | B | A | A |

**[Table 4]**

| | | Example | |
|---|---|---|---|
| | | 14 | 15 |
| Sugar | Raffinose | 1.5 | 1.5 |
| Amino acid | Citrulline | 1.5 | 1.5 |
| Citric acid compound | Citric acid | 0.01 | |
| | Na citrate | | 0.1 |
| pH | Before treatment | 6 | 7 |
| Degree of yellowness | Before treatment | 2.7 | 2.7 |
| | After treatment | 4.8 | 3.5 |
| | Rate of change | 1.8 | 1.3 |
| Evaluation | Coloration suppressing effect | A | A |

Comparative Example 1 comprises, as a sugar, galactose which is a monosaccharide. Comparative Example 2 comprises, as a sugar, a galactooligosaccharide which is a trisaccharide composed of two galactoses and one glucose as constituent sugars. As shown in Table 2, Comparative Example 1 and Comparative Example 2 exhibit a rate of change in degree of yellowness before and after the storage treatment of 20 or more, and are significantly discolored due to the storage treatment.

On the other hand, Examples 1 to 10, 14, and 15 each comprise, as a sugar, raffinose which is a trisaccharide composed of one fructose, one galactose, and one glucose as constituent sugars and is a non-reducing sugar. Examples 11 to 13 each comprise, as a sugar, lactose which is a disaccharide composed of one galactose and one glucose as constituent sugars and is a reducing sugar. As shown in Tables 2 to 4, Examples 1 to 15 exhibit a rate of change in degree of yellowness before and after the storage treatment of 4 or less, and are significantly suppressed in discoloration due to the storage treatment as compared with Comparative Examples 1 and 2 exhibiting a rate of change of 20 or more. From these results, it is understood that the discoloration suppressing effect is obtained by using, as the specific sugar, a polysaccharide including a single galactose as a constituent sugar.

Among the Examples, Examples 1 to 10, 14, and 15 comprising a non-reducing sugar as a sugar exhibit a rate of change in degree of yellowness before and after the storage treatment of 2 or less and a particularly high effect of suppressing the discoloration due to the storage treatment. Similarly, Example 12 and 13 in which the combination of the sugar and the amino acid is an acidic amino acid compound (sodium glutamate or sodium aspartate) and a reducing sugar (lactose) exhibit a rate of change in degree of yellowness before and after the storage treatment of 2 or less and a particularly high effect of suppressing the discoloration due to the storage treatment.

As shown in the results of Examples 1 to 5 and the results of Example 14 and 15, the rate of change in degree of yellowness changes depending on the content of the citric acid compound. From the results, it is understood that the presence of the citric acid compound is important in order to obtain the discoloration suppressing effect according to the Examples.

### S. oralis Culture Test

*S. oralis* was cultured in a BHI medium (Brain Heart Infusion broth (available from BD)). The culture broth was measured for turbidness at 660 nm, and then diluted with a BHI medium to a measured turbidness of 0.1, and this was used as a bacterial liquid. Streptococcus oralis spp. tigurinus was used as *S. oralis.*

Sample solutions corresponding to Examples 1 and 9 to 13, 14, and 15 were prepared. The sample solutions were different from Examples 1 and 9 to 13, 14, and 15 only in that water in the composition shown in Table 1 was changed to a BHI medium.

To a well of a 96-well plate, 100 µl of the bacterial liquid and 100 µl of the sample solution were added, and subjected to culture at 37°C under anaerobic conditions for 24 hours. After the culture, supernatant was removed, and 110 µl of an Alamar Blue solution (available from Invitrogen) diluted 11 times with phosphate buffered saline (PBS) was added, and fluorescence intensity was measured at an excitation wavelength of 545 nm and a fluorescence wavelength of 590 nm.

The relative fluorescence intensity of each measured sample, compared to that of a control to which no sample solution was added, was calculated as a growth degree of *S. oralis.* The result demonstrated thatin all cases where the sample solutions corresponding to Examples 1 and 9 to 13, 14, and 15 were used, the growth degree exceeded 1, indicating a promotion of *S. oralis* proliferation.

## Claims

1. An oral composition comprising a specific sugar, an amino acid, and a citric acid compound, wherein
the specific sugar is a polysaccharide including a single galactose as a constituent sugar,
the citric acid compound is at least one selected from citric acid and a citrate salt,
a content of the specific sugar is 0.5 mass% or more and 5 mass% or less,
a content of the amino acid is 0.5 mass% or more and 4 mass% or less, and
a content of the citric acid compound is 0.001 mass% or more and 2 mass% or less.

2. The oral composition according to claim 1, wherein the specific sugar is a non-reducing sugar.

3. The oral composition according to claim 2, wherein
the amino acid is at least one selected from a basic amino acid compound and an acidic amino acid compound,
the basic amino acid compound is at least one selected from a basic amino acid and a basic amino acid salt, and
the acidic amino acid compound is at least one selected from an acidic amino acid and an acidic amino acid salt.

4. The oral composition according to claim 3, wherein
the basic amino acid compound is arginine,
the acidic amino acid compound is at least one selected from glutamic acid, a glutamate salt, aspartic acid, and an aspartate salt, and
the non-reducing sugar is raffinose.

5. The oral composition according to claim 2, wherein the amino acid is a neutral amino acid.

6. The oral composition according to claim 5, wherein
the neutral amino acid is citrulline, and
the non-reducing sugar is raffinose.

7. The oral composition according to claim 1, wherein
the specific sugar is lactose, and
the amino acid is at least one acidic amino acid compound selected from an acidic amino acid and an acidic amino acid salt.

8. The oral composition according to claim 7, wherein the acidic amino acid compound is at least one selected from glutamic acid, a glutamate salt, aspartic acid, and an aspartate salt.

9. The oral composition according to any one of claims 1 to 7, which is used as an oral composition for bacterial proliferation that promotes proliferation of S. oralis.
